# EUROPEAN PATENT SPECIFICATION

(11) **EP 4 568 648 B1**
(45) Date of publication and mention of the grant of the patent: **04.03.2026**
(21) Application number: 23751595.2
(22) Date of filing: 01.08.2023
(51) Int. Cl.: A61K 8/06, A61K 8/27, A61K 8/81, A61Q 15/00, A61Q 17/00, A61K 8/36, A61K 8/02

(54) **EMULSION DEODORANT COMPOSITIONS**
EMULSIONSDEODORANTZUSAMMENSETZUNGEN
COMPOSITIONS DÉODORANTES SOUS FORME D'ÉMULSION

(30) Priority: 11.08.2022 EP 22189840
(43) Date of publication of application: 18.06.2025
(73) Proprietor: Unilever IP Holdings B.V., 6708 WH Wageningen (NL); Unilever Global IP Limited, Wirral, Merseyside CH62 4ZD (GB)
(72) Inventor: CORNMELL, Robert Joseph, 6708 WH Wageningen (NL); CROSS, Nigel John, 6708 WH Wageningen (NL); EMSLIE, Bruce Steven, 6708 WH Wageningen (NL); JAMES, Alexander Gordon, 6708 WH Wageningen (NL); MCWALTER, Joy Rachel, 6708 WH Wageningen (NL)
(74) Representative: Whaley, Christopher
(86) International application number: PCT/EP2023/071260
(87) International publication number: WO 2024/033154

(56) References cited:
- EP-A1- 3 708 145
- WO-A1-2022/136009
- AU-A1- 2019 259 299
- US-A1- 2020 046 619
- US-B2- 7 138 128
- US-B2- 7 745 425
- GALLARDO V ET AL: "SUNSCREEN FORMULATION: A STUDY OF SILICONE-EMULSIFIANT CONCENTRATION", JOURNAL OF APPLIED COSMETOLOGY, INTERNATIONAL EDIEMME, ROME, IT, vol. 16, no. 2, 1 April 1998 (1998-04-01), pages 37 - 44, XP008021200, ISSN: 0392-8543

## Description

### Field of Invention

The invention is concerned with emulsion deodorant compositions, particularly such compositions having enhanced activity via inhibition of bacteria on the skin surface.

### Background

Emulsion deodorant compositions, including water-in-oil emulsions are known in the art. The format of such compositions may typically be lotions, creams, soft solids or solid sticks. Compositions of the present invention may also take any of these formats.

Deodorant compositions comprising zinc-based actives are known in the art. Examples of publications disclosing such compositions are given below

Zinc ricinoleate is a particularly well known zinc-based deodorant. EP 3878431 A1 (Hyteck, 2021) discloses stable emulsion deodorant compositions comprising zinc ricinoleate.

WO 18/087147 A1 (Givaudan, 2018) discloses deodorant compositions comprising zinc neodecanoate and fragrance.

WO 18/087148 A1 (Givaudan, 2018) discloses deodorant compositions comprising zinc neodecanoate and aluminium chlorohydrate.

Deodorant compositions comprising polyquaternium compounds are also known in the art. Examples of publications disclosing such compositions are given below.

WO 16/012417 A1 (Beiersdorf, 2017) discloses deodorant compositions comprising a range of polyquaternium compounds.

WO 16/012420 A1 (Beiersdorf, 2017) discloses deodorant compositions comprising polyquaternium-6 (a polymeric quaternary ammonium salt of diallyldimethylammonium chloride). US 7138128 B2 discloses emulsion compositions containing zinc oxide and polyquaternium-10.

### Summary of Invention

The invention is concerned with emulsion deodorant compositions comprising deodorant compositions of enhanced activity. The deodorant compositions involved comprise a synergistic blend of a zinc-based deodorant active and a polyquaternium compound.

It is an object of the invention to provide emulsion deodorant compositions having superior efficacy.

It is a further object of the invention to provide emulsion deodorant compositions that have low irritation potential.

It is a further object of the invention to provide emulsion deodorant compositions that have good rheological stability.

In a first aspect of the invention, there is provided an emulsion deodorant composition comprising a dispersed aqueous phase in a continuous oily phase comprising a liquid oil, a deodorant active system and an emulsifier, wherein the deodorant active system comprises zinc neodecanoate and a polyquaternium compound which is polyquaternium-6

In a second aspect of the invention, there is provided a cosmetic method of obtaining a deodorancy benefit comprising the topical application to the surface of the human body of a deodorant composition according to the first aspect of the invention.

### Detailed Description

Herein, features expressed as "preferred" with regard to a particular aspect of the invention should be understood to be preferred with regard to each aspect of the invention (likewise, features expressed as "more preferred", "particularly preferred" or "most preferred"). "Preferred" features aid the delivery of one or more of the objects of the invention.

Herein, preferred features of the invention are particularly preferred when used in combination with other preferred features (likewise, features expressed as "more preferred", "particularly preferred" or "most preferred").

Herein, "ambient conditions" refer to 20°C and 1 atmosphere pressure, unless otherwise indicated.

Herein, water-soluble refers to materials having a solubility in water of at least 10 g/l, such as from 10 g/l to 500 g/l, under ambient conditions.

Herein, all percentages, ratios and amounts are by weight, unless otherwise indicated.

Herein, amounts and concentrations of ingredients are percentages by weight of the total composition, unless otherwise indicated.

Herein, references to molar amounts and ratios of "aluminium" are calculated on the basis of mono-nuclear aluminium, but include aluminium present in poly-nuclear species; indeed, most of the aluminium in the salts of relevance is present in poly-nuclear species.

Herein, references to amounts of components such as "carrier oil" or "thickening agent" relate to the total amount of such components present in the composition.

Herein, the word "comprising" is intended to mean "including" but not necessarily "consisting of", i.e., it is non-exhaustive.

Herein, "cosmetic" methods and compositions should be understood to mean non-therapeutic methods and compositions, respectively.

Compositions of the present invention are water-in-oil emulsions comprising a dispersed aqueous phase in a continuous oily phase comprising a liquid oil, stabilised by an emulsifier. They also comprise a deodorant active system comprising a zinc-based deodorant active and a polyquaternium compound as defined above (*vide infra*)*.*

In some preferred embodiments the emulsion deodorant composition is in the form of a w/o emulsion solidified by the presence of a thickening agent to give an emulsion stick or soft solid. Emulsion sticks according to the invention may be clear, translucent or opaque. Suitable thickening agents are described hereinafter.

Solid emulsion sticks are a particularly preferred format, especially sticks having a hardness of less than 20 mm, preferably less than 15 mm and more preferably less than 10 mm. Such sticks can demonstrate a combination of aesthetically desirable properties, including sensory benefits such as the avoidance of excessive drag on application and sometimes lower visible marks on clothing worn in close proximity to the area of application on the skin.

Herein, the "hardness" of stick and soft solid compositions refers to the depth, in millimetres, that a cone penetrates into a test specimen under fixed conditions, as determined in accordance with the procedures of ASTM Method D217-48, using a Petrotest PNR10 Penetrometer (or equivalent), equipped with an ASTMD2884 plunger (Petrotest Cat. #18-0081 or equivalent, weight =47.5 g) and a 2.5 g aluminium cone, 20° angle with a base diameter of 10 mm, wherein hardness values are reported as an average of 6 replicate measurements.

The aqueous phase typically comprises from 30 to 70% of the total composition. Preferably, it comprises from 40 to 65% and more preferably from 45 to 60% of the total composition.

Components of the aqueous phase include water, typically the polyquaternium (PQ) compound and any other water-soluble components.

The PQ compound is typically a salt and is preferably water-soluble. Preferably, it is also an antimicrobial deodorant active in its own right.

The PQ compound is PQ-6,

Herein, PQ-6, i.e. polyquaternium-6, is polymeric quaternary ammonium salt of diallyldimethylammonium chloride.

The PQ compound is preferably used at a level of from 0.5 to 15%, more preferably from 0.75 to 10% and most preferably from 1 to 7%.

A highly preferred additional component of the aqueous phase is a humectant selected from glycerol, propylene glycol and PEG oligomers of weight average molecular weight of from 180 to 420, of which glycerol is particularly preferred. The humectant is present in the total composition at a preferred level of from 1 to 20%, more preferably from 2 to 12% and most preferably from 3 to 10%, these levels being particularly relevant when the humectant is glycerol.

The oily phase typically comprises from 30 to 70% of the total composition. Preferably, it comprises from 35 to 60% and more preferably from 40 to 55% of the total composition. For the purpose of calculating the amount of the oil phase, the emulsifier is considered to be a component of the oil phase.

Components of the oily phase comprise a liquid oil, typically a structurant or thickening agent, typically the zinc-based deodorant, and typically a fragrance.

Herein, the terms "oil" and "liquid oil" may be used interchangeably and signify a water-insoluble organic material that is liquid at 20°C. Any material having a solubility of less than 0.1g/100g at 20°C is considered to be insoluble.

A preferred oil for use in compositions prepared in accordance with the invention is a fragrance oil, sometimes alternatively called a perfume oil. The fragrance oil may comprise a single fragrance or component more commonly a plurality of fragrance components. Herein, fragrance oils impart an odour, preferably a pleasant odour, to the composition. Preferably, the fragrance oil imparts a pleasant odour to the surface of the human body the composition is applied to the same.

The amount of fragrance oil in the composition is commonly up to 3% advantageously is at least 0.5% and particularly from 0.8% to 2%.

The total amount of liquid oil in the composition is preferably from 5 to 75%, more preferably from 10 to 60%, and most preferably at from 20 to 55% by weight of the total composition.

In certain embodiments, it is preferred to include an oil, other than a fragrance oil, that has a relatively low viscosity, by which is meant less 250 cS (mm².s⁻¹). Such oils can improve the sensory properties of the composition on application and can lead to other benefits such as emolliency.

Suitable oils can be selected from alkyl ether oils having a boiling point of above 100°C and especially above 150°C, including polyalkyleneglycol alkyl ethers. Such ethers desirably comprise between 10 and 20 ethylene glycol or propylene glycol units and the alkyl group commonly contains from 4 to 20 carbon atoms. The preferred ether oils include polypropylene glycol alkyl ethers such as PPG-14-butylether and PPG-15-stearyl ether.

Suitable oils can include one or more triglyceride oils. The triglyceride oils commonly comprise the alkyl residues of aliphatic C₇ to C₂₀ acids, the total number of carbon atoms being selected in conjunction with the extent of olefinic unsaturation and/or branching to enable the triglyceride to be liquid at 20°C. One example is jojoba oil. Particularly preferably, in the triglyceride oil the alkyl residues are linear C₁₈ groups having one, two or three olefinic degrees of unsaturation, two or three being optionally conjugated, many of which are extractable from plants (or their synthetic analogues), including triglycerides of oleic acid, linoleic acid, conjugated linoleic acids, linolenic acid, petroselenic acid, ricinoleic acid, linolenelaidic acid, trans 7-octadecenoic acid, parinaric acid, pinolenic acid, punicic acid, petroselenic acid and stearidonic acid.

Suitable oils can include those derived from unsaturated C₁₈ acids, including coriander seed oil, impatiens balsimina seed oil, parinarium laurinarium kernel fat oil, sabastiana brasilinensis seed oil, dehydrated castor seed oil, borage seed oil, evening primrose oil, aquilegia vulgaris oil, sunflower (seed) oil and safflower oil. Other suitable oils are obtainable from hemp, and maize corn oil. An especially preferred oil by virtue of its characteristics is sunflower (seed) oil.

Further suitable oils, that can also be emollient oils, comprise alkyl or alkyl-aryl ester oils having a boiling point of above 150°C (and a melting point of below 20°C). Such ester oils include oils containing one or two alkyl groups of 12 to 24 carbon atoms length, including isopropyl myristate, isopropyl palmitate and myristyl palmitate. Other non-volatile ester oils include alkyl or aryl benzoates such C₁₂₋₁₅ alkyl benzoate, for example Finsolv TN^{™} or Finsolv Sun^{™},

A further class of suitable oils comprises non-volatile dimethicones, often comprising phenyl or diphenylene substitution, for example Dow Corning 200 350cps or Dow Corning 556.

A preferred component of compositions of the invention is a thickening agent, sometimes alternatively referred to as a structurant, gelling agent or gellant. Such agents increase the viscosity of or solidify the liquid oil in which the disperse aqueous phase is suspended. They are particularly preferred for use in stick compositions.

The thickening agent may be selected from any of those known in the art, including those as described further hereinbelow.

The structurant or agent is preferably a hydrocarbon wax blend, meaning that it consists of various molecules consisting of solely carbon and hydrogen.

Herein, the term "wax blend" or "wax blends" may sometimes be substituted by the term "wax" or "waxes" respectively, without change of meaning.

Preferred hydrocarbon waxes are polyolefin waxes, in particular polyethylene waxes. Such waxes are typically synthetic waxes, i.e. waxes synthesised from olefins in the case of polyolefin waxes and waxes synthesised from ethylene in the case of polyethylene waxes.

Preferred hydrocarbon wax blends that may be used in the present invention have an average molecular weight (M_{w}) of from 450 to 500 and particularly from 460 to 490. Preferred hydrocarbon wax blends may also independently have a polydispersity (M_{w}/Mₙ) of from 1.01 to 1.05, particularly 1.02 to 1.04. A combination of either of the above preferred average molecular weight ranges may be employed with either of the preferred polydispersity ranges to advantageous effect.

The amount of the structurant or thickening agent used in stick compositions of the invention is preferably from 5%, more preferably from 7% and most preferably from 8%. The upper amount is preferably up to 25%, more preferably up to 20% and most preferably up to 15%. The preferred ranges of solidifying agent may be selected from any of the lower and upper amounts indicated in this paragraph.

The zinc-based deodorant active is a hydrophobic material, in particular a zinc carboxylate. Such zinc carboxylates may often be zinc dicarboxylates, reflecting the stoichiometry of salts formed between divalent zinc ions and monovalent carboxylates. Herein, references to zinc carboxylates also include zinc dicarboxylates.

Preferred zinc carboxylates salts are salts of a fatty acid having from 6 to 18 carbon atoms, in particular zinc dicarboxylates salts of a fatty acid having from 6 to 18 carbon atoms. Such salts are relative hydrophobic in nature, enhancing the ease with which they can be formulated with other hydrophobic components of the oily phase of the emulsion.

To enhance the hydrophobicity of the C6-C18 fatty acid, preferred fatty acids are selected from those having few hydrophilic groups, notably less than two hydroxyl groups per fatty acid residue. Hence, particularly preferred zinc carboxylates are zinc dicarboxylates of a C6 to C18 fatty acid having less than two hydroxy groups per fatty acid residue.

Preferred zinc carboxylate salt used in the present invention are salts of a fatty acid having from 8 to 14 carbon atoms, particularly 8 to 12 carbon atoms.

Preferred zinc carboxylate salts used in the present invention are salts of a saturated fatty acid.

Preferred zinc carboxylate salts used in the present invention are salts of a branched chain fatty acid, particularly when having from 8 to 12 carbon atoms and more particularly when saturated.

The zinc carboxylate used in the present invention is zinc neodecanoate, which is a salt of zinc and neodecanoic acid having the formula [C₉H₂₀-CO.O]₂Zn.

The zinc-based deodorant active is preferably present at from 0.1 to 20%, more preferably at from 0.5 to 10% and most preferably at from 1 to 7% of the total composition; these figures being particularly relevant when the zinc-based active is a zinc carboxylate salt.

The ratio of the zinc-based deodorant active to the PQ compound is preferably from 1: 6 to 6: 1, more preferably from 1: 4 to 4: 1 and most preferably from 1: 2 to 2: 1; these ratios being particularly relevant when the zinc-based active is a zinc carboxylate salt and especially relevant when the zinc-based active is a zinc carboxylate salt and the PQ compound is PQ-6.

The total proportion of emulsifiers in the composition is preferably at least 0.5% and particularly at least 1% by weight. Commonly, the emulsifiers are not present at above 10%, often not more than 7% by weight and in many preferred embodiments up to 6% by weight. An especially desirable concentration range for the emulsifiers is from 1 to 5% by weight.

It is preferred that compositions of the invention comprise a non-ionic emulsifier system. Such an emulsifier system preferably has a mean HLB value in the region of from 1 to 6 and particularly from 1 to 5. An especially desired mean HLB value is from 3 to 5. Such a mean HLB value can be provided by selecting an emulsifier having such an HLB value, or by employing a combination of at least two emulsifiers, such that the weighted mean HLB is within the indicated ranges.

An especially desirable range of emulsifiers comprises a hydrophilic moiety provided by a polyalkylene oxide (polyglycol), and a hydrophobic moiety provided by an aliphatic hydrocarbon, preferably containing at least 10 carbons and commonly linear. The hydrophobic and hydrophilic moieties can be linked via an ester or ether linkage, possibly via an intermediate polyol such as glycerol. A preferred range of emulsifiers comprises polyethylene glycol ethers.

In composition comprises a silicone oil, an alkyl dimethicone copolyol is a preferred type of emulsifier. Such compositions are preferably w/o emulsion sticks or soft solids.

A preferred additional component of compositions of the invention is a vitamin, such as vitamin B (particularly vitamin B3). Vitamins are preferably included at a total level of from 0.1 to 10% and more preferably at from 0.5 to 7.5%.

Other components that may be included in compositions according to the invention including those described in the following paragraphs.

Wash-off agents may be included, often in an amount of up to 10%, to assist in the removal of the formulation from skin or clothing. Such wash-off agents are typically non-ionic surfactants such as esters or ethers containing a C₈ to C₂₂ alkyl moiety and a hydrophilic moiety comprising a polyoxyalkylene group (POE or POP).

Skin feel improvers (e.g. talc or finely divided high molecular weight polyethylene), may be included, typically in an amount from 1 up to 10%.

Skin moisturisers, such as glycerol or polyethylene glycol (e.g. mol. wt. 200 to 600) may be included, typically in an amount of up to 5%.

Skin benefit agents, such as allantoin or lipids, may be included, typically in an amount of up to 5%.

A highly preferred optional component is a preservative system, such as sodium benzoate/citric acid and/or an antioxidant such BHT (butyl hydroxy toluene), each typically in an amount of from 0.01 to 0.5%.

Other components that may be present include short chain (C₂-C₄) alcohols and especially polyols such glycerol, ethylene glycol, propylene glycol and polymers thereof, in particular poly(ethylene glycol) and poly(propylene glycol). Poly(ethylene glycol) of average molecular weight 200 to 600 is a preferred component. Such components may add to the sensory properties of the composition and, when included, are typically present at from 0.5 to 10% of the total composition.

Compositions of the invention which are stick compositions may be prepared by any of methods known in the art. Two preferred methods are described hereinbelow as the "hot process" and the "cold process". Of these the "hot process" is particularly preferred for mass production as this allows easier factory processing of the zinc-based deodorant active, particularly when this is a zinc carboxylate and especially when this is zinc neodecanoate.

The "hot process" proceeds as follows: in step 1, making an aqueous phase by blending together water and water-soluble ingredients, including the polyquaternium compound; in step 2, forming an oil phase comprising the thickening agent or structurant and the liquid oil or oil mixture, which normally entails heating the thickening agent or structurant and oils to a temperature at which thickening agent or structurant particles are no longer visible, commonly in the region of the melting point of the said thickening agent or structurant; and in a step 3, mixing the aqueous phase and the oil phase together at elevated temperature such that the thickening agent or structurant remains in a liquid state dissolved in the liquid oil, and preferably shear mixing the two phases, in the presence of the emulsifier, and in step 4, filling the resultant mixture into a dispenser whilst it is still mobile and thereafter cooling or allowing the composition to cool, initially until the mixture solidifies and then attains ambient temperature.

Steps 1 and 2 in the process described in the paragraph immediately above can be carried out simultaneously or sequentially. In step 2, the oil is often heated to a temperature in the region of 70 to 80°C, preferably 70 to 75°C. Before the aqueous phase, prepared in step 1, is mixed with the oil phase in step 3, it preferably heated to a temperature in the region of 60 to 70°C, more preferably 65 to 70°C and most preferably 68 to 69°C. Using these elevated temperatures for the aqueous phase can aid the emulsification process. During step 3, the temperature is maintained at preferably 65 to 80°C, more preferably 65 to 75°C and most preferably 66 to 74°C.

The process described in the paragraphs above may be performed by batch or continuous (including semi-continuous) processes.

The "cold process" proceeds as follows: (i) making an aqueous phase by blending together water and a polyquaternium compound; (ii) forming an oil phase; (iii) mixing the aqueous phase and the oil phase together, preferably at high shear, to form an emulsion at ambient temperature in the presence of an emulsifier; (iv) adding a thickening agent or structurant, in solid form, to the emulsion formed in step (iii) and heating the mixture to a temperature of 70°C or greater in order to melt/dissolve the thickening agent or structurant; (v) filling the resultant mixture into a dispenser whilst it is still mobile and (vi) cooling the composition until the mixture solidifies.

### Examples

Example 1 and Comparative Examples A, B and C indicated in Table 1 were prepared as liquid water-in-oil emulsions by methods known in the art.

The following raw materials were used in the Examples:
Polarswachs N670: Synthetic wax MP 70 BHT, structurant, a synthetic polyethylene wax having a melting point of 71°C, ex Morre-Tec Industries, NJ.
DC245: cyclopentasiloxane, ex Dow.
Finsolv TN: C12-15 alkyl benzoate, ex Innospec.
SSO: "sunflower seed oil", *Helianthus annuus,* ex Akosun.
Zinc-based (deo.) RM: a raw material comprising zinc neodecanoate (80%), isopropyl myristate (19%) and fragrance (1%).
Abil EM90: silicone-based emulsifier, cetyl PEG/PPG-10 dimethicone, ex Evonik.
Glycerol: Pricerine 9091, ex Croda.
Aqua: water.
Merquat 100: Polyquaternium-6 (PQ-6), poly(diallyldimethylammonium chloride), 43% aqueous solution, ex Lubrizol. Amounts indicated in Tables 1 and 5 are of this solution.
Merquat 295: Polyquaternium-22 (PQ-22), 2-propen-1-aminium, N,N-dimethyl-N-2-propenyl-, chloride, polymer with 2-propenoic acid, 35-40% aqueous solution, ex Lubrizol. Amounts indicated in Table 5 are of this solution.
Vitamin B3: Niacinamide Pharmacopoeia Grade, ex Veer Chemie.
Citric acid: ex Jungbunzlauer.
Fragrance: a blend of several perfume notes and solvent(s).

**Table 1: Compositions**

| | **Examples** | | | |
|---|---|---|---|---|
| Components | **A** | **B** | **C** | **1** |
| | Parts by weight | | | |
| Polarswachs N670 | -- | -- | -- | -- |
| DC245 | 19 | 19 | 19 | 19 |
| Finsolv TN | 13 | 13 | 11 | 9 |
| SSO | -- | -- | -- | -- |
| Zinc-based deo. RM | -- | -- | 2 | 2 |
| Abil EM90 | 1.2 | 1.2 | 1.2 | 1.2 |
| Glycerol | 18 | 18 | 18 | 18 |
| Aqua | 32.2 | 34.5 | 36.8 | 36.8 |
| Merquat 100 | 4.6 | -- | -- | 4.6 |

The antimicrobial activity of the compositions described in Table 1 was determined using an *in-vitro* model for bacterial growth on human stratum corneum, based on that described by van der Krieken *et al.* (2016)*. The assay utilises human foot callus as a physical substrate and nutrient source for the growth of the test bacterium, *Staphylococcus hominis* J23.

Callus was collected from healthy volunteers using an electronic foot file in full accordance with the Declaration of Helsinki and UK Human Tissue Act 2004. Collected material was pooled into a single combined sample, frozen in liquid nitrogen, and ground into a fine powder using a mortar and pestle. This was accurately weighed into ~20 mg aliquots which were sterilised by gamma-irradiation prior to storage at -20°C until required. Prior to use, each aliquot was suspended in phosphate-buffered saline to a final concentration of 20 g/l.

For the assay set-up, 1 ml of 10 g/l agarose was initially dispensed into each well of a 24-well microtitre plate and left to dry overnight in a microbiological safety cabinet. Wells were then loaded with 75 ml callus suspension (thoroughly agitated beforehand, and spread uniformly) and left to dry for a further 3 hr. The bacterial inoculum was prepared from a fresh overnight culture of *S. hominis* grown on Tryptone Soya Agar, resuspended in normal saline to an optical density (OD₆₂₀) of ~0.8, equivalent to -10⁸ colony-forming units (CFU) per ml. 40 ml of this bacterial suspension was then added to each well of the microtitre plate (apart from the cell-free controls). The test formulations** (30 ml per well) were next added to the plate (apart from the cells-only and cell-free controls), with each treatment (and control) run in quadruplicate. Finally, 25 ml resazurin solution (1 g/l) was added to each well, and the microtitre plate agitated using a magnetic shaker, prior to incubation at 37°C for 48 hr.

Fluorescence measurements (excitation at 545 nm; emission at 590 nm) were recorded at 24 hr. and 48 hr. using a microplate reader, and the mean value calculated from the four replicates for each treatment and control. These are referred to as "F values" in the tables below. The assay works on the principle that the irreversible reduction of weakly fluorescent resazurin to the highly fluorescent resorufin is proportional to the level of aerobic respiration, and thus growth by metabolically active bacteria.
* van der Krieken, et al. An In vitro Model for Bacterial Growth on Human Stratum Corneum. Acta Derm. Venereol. 96, 873-879 (2016).
   ** As indicated in Table 1, but diluted to 25% original concentration with phosphate-buffered saline.

The results are given in Tables 2 to 4 and described further hereinbelow.

After 24 hrs., Comparative Example A [with 4.6% Merquat 100 (equivalent to 2.0% PQ-6)] gave a very small effect on bacterial growth inhibition - 75% growth relative to the "just bacteria" control.

After 24 hrs., Comparative Example C [with 2% zinc-based deodorant active (equivalent to 1.6% zinc neodecanoate)] gave a small effect on bacterial growth - 40% growth relative to the "just bacteria" control.

After 24 hrs., Example 1 [with 4.6% Merquat 100 (2% PQ-6) and 2% zinc-based deodorant active (1.6% zinc neodecanoate)] gave a large reduction in bacterial growth - only 8% growth relative to the "just bacteria" control. The ratio of zinc neodecanoate to PQ-6 in Example 1 was 1: 1.25.

After 48 hrs., Comparative Example A again gave 75% growth; Comparative Example C gave 38% growth; and Example 1 gave only 7% growth, each relative to the "just bacteria" control.

**Table 2: Callus Assay Results**

| **Examples** | **F Value Time** | |
|---|---|---|
| | After 24 hrs. | After 48 hrs. |
| Just bacteria | 1152 | 1385 |
| **A** | 859 | 1091 |
| **B** | 855 | 1027 |
| **C** | 457 | 532 |
| **1** | 93 | 91 |
| Without bacteria | 50 | 61 |

The results for Example 1 are indicative of synergistic bacterial growth inhibition by the combination of Merquat 100 (PQ-6) and zinc neodecanoate.

Table 3 illustrates the same results relative to Comparative Example B, a control emulsion sample lacking both Merquat and zinc neodecanoate.

**Table 3: Callus Assay Results relative to Comparative Example B**

| **Examples** | **Relative F value (%)** | |
|---|---|---|
| | After 24 hrs. | After 48 hrs. |
| Just bacteria | 135 | 135 |
| **A** | 100 | 106 |
| **B** | 100* | 100* |
| **C** | 53 | 52 |
| **1** | 10.5 | 9 |

| | | |
|---|---|---|
| * By definition. | | |

The results from the callus assay may alternatively expressed as percent inhibition of bacterial growth. This expression of the results is shown in Table 4. As a first step in calculating these results, the dynamic range at each timepoint was determined by subtracting the F-value for the "without bacteria" control from the "just bacteria" control. Then an "adjusted F-value" for each assay was calculated by subtracting the "without bacteria" F-value from the actual F-value. Finally, the percent inhibition [of bacterial growth] was calculated as "adjusted f-value" as a percentage of the dynamic range.

As can be seen, Example 1 gave a surprisingly high inhibition of bacterial growth in this study, indicative of synergistic bacterial growth inhibition by the combination of Merquat 100 (PQ-6) and zinc neodecanoate.

**Table 4: Callus Assay Results as Percent Inhibition of Bacterial Growth**

| **Examples** | **Percent Inhibition (%)** | |
|---|---|---|
| | After 24 hrs. | After 48 hrs. |
| Just bacteria | 0 | 0 |
| **A** | 24 | 24 |
| **B** | 24 | 19 |
| **C** | 61 | 62 |
| **1** | 95 | 97 |

Table 5 gives details of further compositions according to the invention.

**Table 5: Further Compositions**

| | **Examples** | | | | |
|---|---|---|---|---|---|
| Components | **2** | **3** | **4** | **5** | **6** |
| | Parts by weight | | | | |
| Polarswachs N670 | 12 | -- | -- | 12 | 12 |
| Polyethylene 400 BHT Prill | -- | -- | -- | -- | 1 |
| DC245 | 14 | 21.1 | 20.6 | -- | 19 |
| Liquid alkane mix | -- | -- | -- | 12 | -- |
| Finsolv TN | 9 | 14.4 | 14.1 | 5 | 9 |
| SSO | 0.1 | -- | -- | 0.1 | 0.1 |
| Zinc-based deo. RM | 4 | 2.2 | 4.3 | 2 | 2 |
| Abil EM90 | 1.2 | 1.3 | 1.3 | 0.8 | 1.2 |
| Fragrance | 1.1 | -- | -- | 1.05 | 1 |
| Glycerol | 18 | 20 | 19.5 | 18 | 12 |
| Aqua | 36 | 38.2 | 34.8 | 40.8 | 32.45 |
| Merquat 100 | 4.6 | - | - | 4.6 | 4.6 |
| Merquat 295 | -- | 2.8 | 5.4 | -- | -- |
| Vitamin B3 | -- | -- | -- | 1 | 5 |
| Sodium benzoate | -- | -- | -- | 0.3 | 0.3 |
| Citric acid | -- | -- | -- | 0.1 | 0.1 |

Examples 2, 5 and 6 were prepared by a process involving the following steps:
(i) making an aqueous phase by blending together water, Merquat 100, and glycerol;
(ii) forming an oil phase comprising the zinc-based deodorant raw material, the oil components and the emulsifier;
(iii) mixing the aqueous phase and the oil phase together at high shear, to form an emulsion at ambient temperature;
(iv) adding a solidifying agent, in solid form, to the emulsion formed in step (iii) and heating the mixture to a temperature of 70°C or greater in order to melt/dissolve the solidifying agent;
(v) filling the resultant mixture into a dispenser whilst it is still mobile and
(vi) cooling the composition until the mixture solidifies.

The other examples (not according to the invention) were prepared by methods known in the art.

In Example 2, the ratio of zinc neodecanoate to PQ-6 was 1.6: 1.

In Example 3, the ratio of zinc neodecanoate to PQ-22 was ca. 1.7: 1.

In Example 4, the ratio of zinc neodecanoate to PQ-22 was ca. 1.7: 1.

In Examples 5 and 6, the ratio of zinc neodecanoate to PQ-6 was 1: 1.25.

## Claims

1. An emulsion deodorant composition comprising (i) a dispersed aqueous phase in a continuous oily phase comprising a liquid oil; (ii) a deodorant active system and (iii) an emulsifier; wherein the deodorant active system comprises zinc neodecanoate and a polyquaternium compound which is polyquaterium-6.

2. An emulsion deodorant composition according to claim 1 comprising a thickening agent or structurant.

3. An emulsion deodorant composition according to claim 2 in the form of a stick or soft solid.

4. An emulsion deodorant composition according to any one of the preceding claims, wherein the emulsifier is selected from nonionic emulsifiers.

5. An emulsion deodorant composition according to any one of the preceding claims which is free from cyclopentasiloxane.

6. An emulsion deodorant composition according to any one of preceding claims, comprising a fragrance.

7. A cosmetic method of obtaining a deodorancy benefit comprising the topical application to the surface of the human body of a deodorant composition according to any one of the preceding claims.

## Patentansprüche

1. Emulsionsdeodorantzusammensetzung, umfassend (i) eine dispergierte wässrige Phase in einer kontinuierlichen öligen Phase, die ein flüssiges Öl umfasst; (ii) ein Deodorant-Wirkstoffsystem und (iii) einen Emulgator; wobei das Deodorant-Wirkstoffsystem Zinkneodecanoat und eine Polyquaterniumverbindung, die Polyquaternium-6 ist, umfasst.

2. Emulsionsdeodorantzusammensetzung nach Anspruch 1, umfassend ein Verdickungsmittel oder Strukturmittel.

3. Emulsionsdeodorantzusammensetzung nach Anspruch 2 in Form eines Stifts oder eines weichen Feststoffs.

4. Emulsionsdeodorantzusammensetzung nach irgendeinem der vorhergehenden Ansprüche, wobei der Emulgator unter nichtionischen Emulgatoren ausgewählt ist.

5. Emulsionsdeodorantzusammensetzung nach irgendeinem der vorhergehenden Ansprüche, die frei von Cyclopentasiloxan ist.

6. Emulsionsdeodorantzusammensetzung nach irgendeinem der vorhergehenden Ansprüche, die einen Duftstoff enthält.

7. Kosmetisches Verfahren zur Erzielung einer desodorierenden Wirkung, umfassend das topische Auftragen einer Deodorantzusammensetzung nach irgendeinem der vorhergehenden Ansprüche auf die Oberfläche des menschlichen Körpers.

## Revendications

1. Composition déodorante en émulsion comprenant (i) une phase aqueuse dispersée dans une phase huileuse continue comprenant une huile liquide; (ii) un système actif déodorant et (iii) un émulsifiant; dans laquelle le système actif déodorant comprend du néodécanoate de zinc et un composé de polyquaternium qui est le polyquaternium-6.

2. Composition déodorante en émulsion selon la revendication 1, comprenant un agent épaississant ou un structurant.

3. Composition déodorante en émulsion selon la revendication 2, sous forme de stick ou de solide mou.

4. Composition déodorante en émulsion selon l'une quelconque des revendications précédentes, dans laquelle l'émulsifiant est choisi parmi les émulsifiants non ioniques.

5. Composition déodorante en émulsion selon l'une quelconque des revendications précédentes, qui est exempte de cyclopentasiloxane.

6. Composition déodorante en émulsion selon l'une quelconque des revendications précédentes, comprenant un parfum.

7. Procédé cosmétique d'obtention d'un effet déodorant, comprenant l'application topique sur la surface du corps humain d'une composition déodorante selon l'une quelconque des revendications précédentes.
